# EUROPÄISCHE PATENTSCHRIFT

(11) **EP 0 415 065 B1**
(45) Veröffentlichungstag und Bekanntmachung des Hinweises auf die Patenterteilung: **28.06.1995**
(21) Anmeldenummer: 90114008.7
(22) Anmeldetag: 21.07.1990
(51) Int. Cl.: C07D 311/68, C07D 405/04, C07D 405/12, A61K 31/44

(54) **Chromanderivate**
Chroman derivatives
Dérivés de chromane

(30) Priorität: 05.08.1989 DE 3926001
(43) Veröffentlichungstag der Anmeldung: 06.03.1991
(73) Patentinhaber: MERCK PATENT GmbH, D-64271 Darmstadt (DE)
(72) Erfinder: Gericke, Rolf, Dr., D-6104 Seeheim (DE); Baumgarth, Manfred, Dr., D-6100 Darmstadt (DE); Lues, Ingeborg, Dr., D-6100 Darmstadt (DE); Bergmann, Rolf, Dr., D-6101 Reichelsheim (DE); De Peyer, Jacques, Dr., CH-3007 Bern (CH)

(56) Entgegenhaltungen:
- EP-A- 0 172 352
- EP-A- 0 250 077
- EP-A- 0 273 262
- GB-A- 2 204 868

## Beschreibung

Die Erfindung betrifft neue Chromanderivate der Formel I 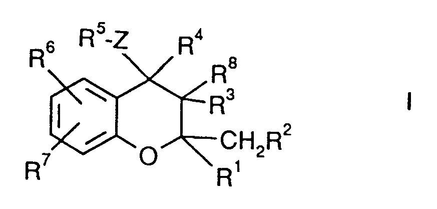
worin
- R¹: A,
- R²: OA,
- R³: OH,
- R⁴: H oder
- R³ und R⁴: zusammen auch eine Bindung,
- R⁵: einen unsubstituierten oder einfach durch A substituierten Oxo-dihydro-pyridyl-rest,
- R⁶: CN,
- R⁷ und R⁸: jeweils H,
- Z: O, NH oder eine Bindung,
- A: Alkyl mit 1-6 C-Atomen,
bedeuten,
sowie deren Salze.

Der Erfindung lag die Aufgabe zugrunde, neue Verbindungen mit wertvollen Eigenschaften aufzufinden, insbesondere solche, die zur Herstellung von Arzneimitteln verwendet werden können.

Es wurde gefunden, daß die Verbindungen der Formel I und ihre physiologisch unbedenklichen Salze bei guter Verträglichkeit wertvolle pharmakologische Eigenschaften besitzen. So zeigen sie Wirkungen auf das cardiovaskuläre System, wobei in der Regel bei niedrigeren Dosen ein selektiver Angriff am Coronarsystem, bei höheren ein blutdrucksenkender Effekt beobachtet werden kann. Am Coronarsystem treten z.B. Widerstandsabnahme und Flußzunahme auf, wobei der Einfluß auf die Herzfrequenz gering bleibt. Weiterhin zeigen die Verbindungen eine relaxierende Wirkung auf verschiedene glattmuskuläre Organe (Gastrointestinaltrakt, Respirationssystem und Uterus). Die Wirkungen der Verbindungen können mit Hilfe an sich bekannter Methoden ermittelt werden, wie sie z.B. in der EP-A1-76075, der EP-A1-173848 oder der AU-A-45547/85 (Derwent Farmdoc Nr. 86081769) sowie von K.S. Meesmann et al., Arzneimittelforschung 25 (11), 1975, 1770-1776, angegeben sind. Als Versuchstiere eignen sich z.B. Mäuse, Ratten, Meerschweinchen, Hunde, Katzen, Affen oder Schweine.

Die Verbindungen können daher als Arzneimittelwirkstoffe in der Human- und Veterinärmedizin verwendet werden. Ferner können sie als Zwischenprodukte zur Herstellung weiterer Arzneimittelwirkstoffe verwendet werden.

In den angegebenen Formeln bedeutet A eine vorzugsweise unverzweigte Alkylgruppe mit 1-6, bevorzugt 1-4, insbesondere 1, 2 oder 3 C-Atomen, im einzelnen vorzugsweise Methyl, ferner bevorzugt Ethyl, Propyl, Isopropyl, Butyl, Isobutyl, weiterhin bevorzugt sek.-Butyl, tert.-Butyl, Pentyl, Isopentyl (3-Methylbutyl), Hexyl oder Isohexyl (4-Methylpentyl).

R¹ ist A, insbesondere Methyl oder Ethyl, bevorzugt Methyl.

R² ist OA, insbesondere Methoxy oder Ethoxy.

Falls R⁴ H bedeutet, ist R³ OH.

Z bedeutet vorzugsweise O oder eine Bindung.

Die Gruppe R⁵-Z ist vorzugsweise 1,2-Dihydro-2-oxo-1-pyridyl, ferner 2-Hydroxy-4-pyridyl-oxy (= 1,2-Dihydro-2-oxo-4-pyridyl-oxy).

Der Rest R⁶ steht vorzugsweise in 6- oder 7-Stellung des Chromansystems.

Von den Resten R⁶ und R⁷ ist R⁷ H, während R⁶ CN ist.

Der Rest R⁸ ist H.

Dementsprechend sind Gegenstand der Erfindung insbesondere diejenigen Verbindungen der Formel I, in denen mindestens einer der genannten Reste eine der vorstehend angegebenen bevorzugten Bedeutungen hat. Einige bevorzugte Gruppen von Verbindungen können durch die nachstehenden Formeln Ia bis Ig ausgedrückt werden, die der Formel I entsprechen und worin die nicht näher bezeichneten Reste die bei der Formel I angegebene Bedeutung haben, worin jedoch
in Ia
   - R¹: A und
   - R²: OA bedeuten;
in Ib
   - R¹: CH₃ und
   - R²: OA bedeuten;
in Ic
   - R¹: CH₃ und
   - R²: OCH₃ bedeuten;
in Id
   - R⁵-Z: 1,2-Dihydro-2-oxo-1-pyridyl oder
   2-Hydroxy-4-pyridyl-oxy bedeuten;
in Ie
   - R⁵-Z: 1,2-Dihydro-2-oxo-1-pyridyl bedeutet;
in If
   - R¹: CH₃,
   - R²: OCH₃,
   - R⁵-Z: 1,2-Dihydro-2-oxo-1-pyridyl oder
   2-Hydroxy-4-pyridyl-oxy
   - R⁸: H bedeuten;
in Ig
   - R¹: CH₃,
   - R²: OCH₃,
   - R⁵-Z: 1,2-Dihydro-2-oxo-1-pyridyl und
   - R⁸: H oder CH₃ bedeuten.

Weiterhin sind bevorzugt Verbindungen der Formeln I' sowie Ia' bis Ig', die den Formeln I sowie Ia bis Ii entsprechen, worin jedoch jeweils zusätzlich R³ OH und R⁴ H bedeuten.

Weiterhin sind bevorzugt Verbindungen der Formeln I'' sowie Ia'' bis Ig'', die den Formeln I sowie Ia bis Ig entsprechen, worin jedoch jeweils zusätzlich R³ und R⁴ zusammen eine Bindung bedeuten.

Im übrigen haben vor- und nachstehend die Reste R¹ bis R⁸, Z und A die bei Formel I angegebenen Bedeutungen, wenn nicht ausdrücklich etwas anderes angegeben ist.

Gegenstand der Erfindung ist ferner ein Verfahren zur Herstellung von Chromanderivaten der Formel I, dadurch gekennzeichnet, daß man ein Chroman der Formel II 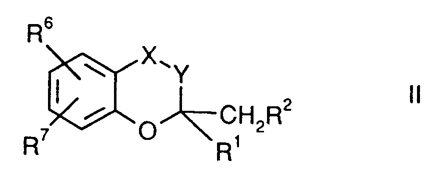
worin
- X-Y: 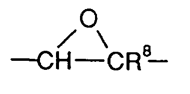 oder -CHE-CR³R⁸- und
- E: Cl, Br, J oder eine reaktionsfähige veresterte OH-Gruppe bedeuten und
- R¹, R², R³, R⁶, R⁷ und R⁸: die bei Formel I angegebenen Bedeutungen haben,
mit einer Verbindung der Formel III

R⁵-Z-H III

worin
- R⁵ und Z: die bei der Formel I angegebenen Bedeutungen haben,
umsetzt,
und/oder daß man eine Verbindung der Formel I, worin R³ OH und R⁴ H bedeutet, dehydratisiert und/oder daß man in einer Verbindung der Formel I einen oder mehrere der Reste R², R³, R⁵ und/oder R⁶ in andere Reste R², R³, R⁵ und/oder R⁶ umwandelt und/oder daß man eine basische Verbindung der Formel I durch Behandeln mit einer Säure in eines ihrer Säureadditionssalze umwandelt.

Die Verbindungen der Formel I werden im übrigen nach an sich bekannten Methoden hergestellt, wie sie in der Literatur (z.B. in den Standardwerken wie Houben-Weyl, Methoden der organischen Chemie, Georg-Thieme-Verlag, Stuttgart; Organic Reactions, John Wiley & Sons, Inc., New York; sowie in den oben angegebenen Patentanmeldungen) beschreiben sind, und zwar unter Reaktionsbedingungen, die für die genannten Umsetzungen bekannt und geeignet sind. Dabei kann man auch von an sich bekannten, hier nicht näher erwähnten Varianten Gebrauch machen.

Die Ausgangsstoffe können gewünschtenfalls auch in situ gebildet werden, derart, daß man sie aus dem Reaktionsgemisch nicht isoliert, sondern sofort weiter zu den Verbindungen der Formel I umsetzt.

Vorzugsweise werden die Verbindungen der Formel I durch Reaktion von Verbindungen der Formel II mit Verbindungen der Formel III hergestellt, zweckmäßig in Gegenwart eines inerten Lösungsmittels bei Temperaturen zwischen etwa 0 und 150°.

Ausgangsstoffe der Formel II mit 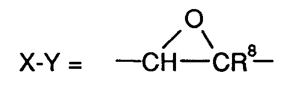
(3,4-Epoxychromane) sind bevorzugt.

Die Ausgangsstoffe III sind in der Regel bekannt (vgl. z.B. DE-OS 37 26 261). Sofern sie nicht bekannt sind, können sie nach an sich bekannten Methoden hergestellt werden. Ausgangsstoffe der Formel II 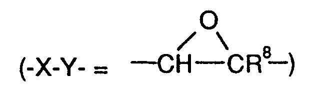
sind erhältlich durch Umsetzung von 2-Hydroxyacetophenonen der Formel 2-HO-R⁶R⁷C₆H₂-COCH₃ mit Ketonen der Formel R¹-CO-CH₂R² zu entsprechenden 4-Chromanonen der Formel IVa, 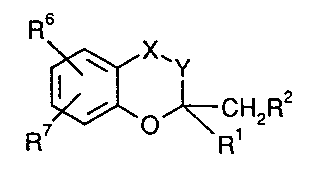
IVa -X-Y- = -CO-CH₂-
IVb -X-Y- = -CO-C(=CH-R¹⁰)-
IVc -X-Y- = -CHOH-CHR⁸-
IVd -X-Y- = -CH=CR⁸-
IVe -X-Y- = -CHBr-CR⁸OH-
gegebenenfalls Kondensation mit Aldehyden der Formel R¹⁰-CHO (R¹⁰ = Alkyl mit 1-5 C-Atomen) zu 3-Alkyliden-4-chromanonen der Formel IVb, Reduktion z.B. mit NaBH₄ zu Chromanolen der Formel IVc, Dehydratisierung z.B. mit p-Toluolsulfonsäure zu Chromenen der Formel IVd und Oxydation, z.B. mit 3-Chlorperbenzoesäure. die letztgenannte Oxydation kann auch mehrstufig erfolgen. So kann man z.B. mit N-Bromsuccinimid in wäßriger Lösung zunächst die Bromhydrine der Formel IVe herstellen und aus diesen anschließend mit einer Base, z.B. Natronlauge, HBr abspalten.

In Verbindungen der Formel II (-X-Y- = -CHE-CR³R⁸-) kommen als "reaktionsfähig veresterte OH-Gruppen" insbesondere die Ester mit Alkylsulfonsäuren (worin die Alkylgruppe 1-6 C-Atome enthält) oder mit Arylsulfonsäure (worin die Arylgruppe 6-10 C-Atome enthält) in Betracht. Diese Verbindungen sind erhältlich aus den 4-Chromanolen der Formel IVc durch Umsetzung mit einem anorganischen Säurehalogenid wie PCl₃, PBr₃, SOCl₂ oder SOBr₂ oder mit einem Sulfonsäurechlorid wie Methan- oder p-Toluolsulfonsäurechlorid.

Bei der Umsetzung von II mit III ist es zweckmäßig, in Gegenwart einer Base zu arbeiten. Als Basen eignen sich bevorzugt Alkalimetall-alkyle, insbesondere Methyllithium oder Butyllithium, Alkalimetall-dialkylamide, insbesondere Lithiumdiisopropylamid, weiterhin z.B. Alkalimetall- oder Erdalkalimetall-hydroxide, -carbonate, -alkoholate, -hydride oder auch -amide wie NaOH, KOH, Ca(OH)₂, Na₂CO₃, K₂CO₃, Na- oder K-methylat, -ethylat oder -tert.-butylat, NaH, KH, CaH₂, NaNH₂, KNH₂, ferner organische Basen wie Triethylamin oder Pyridin, die auch im Überschuß angewendet werden können und dann gleichzeitig als Lösungsmittel dienen.

Als inerte Lösungsmittel eignen sich insbesondere Alkohole wie Methanol, Ethanol, Isopropanol, n-Butanol oder tert.-Butanol; Ether wie Diethylether, Diisopropylether, Tetrahydrofuran (THF) oder Dioxan; Glykolether wie Ethylenglykolmonomethyl- oder -monoethylether (Methylglykol oder Ethylglykol), Ethylenglykoldimethylether (Diglyme); Ketone wie Aceton oder Butanon; Nitrile wie Acetonitril; Nitroverbindungen wie Nitromethan oder Nitrobenzol; Ester wie Ethylacetat; Amide wie Dimethylformamid (DMF), Dimethylacetamid oder Phosphorsäurehexamethyltriamid; Sulfoxide wie Dimethylsulfoxid (DMSO); chlorierte Kohlenwasserstoffe wie Dichlormethan, Chloroform, Trichlorethylen, 1,2-Dichlorethan oder Kohlenstofftetrachlorid; Kohlenwasserstoffe wie Benzol, Toluol oder Xylol. Weiterhin eignen sich Gemische dieser Lösungsmittel untereinander.

Das Epoxid II 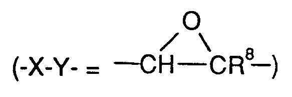
kann auch in situ hergestellt werden,. z.B. durch Einwirkung einer Base auf das entsprechende Bromhydrin IVe.

Eine besonders bevorzugte Arbeitsweise besteht darin, daß äquivalente Mengen der Ausgangsstoffe der Formeln II und III in einem Alkohol wie Ethanol in Gegenwart einer Base wie Pyridin einige Zeit kocht.

Eine Verbindung der Formel I, worin R³ = OH und R⁴ = H ist, kann durch Behandeln mit einem Dehydratisierungsmittel in eine Verbindung der Formel I, worin R³ und R⁴ zusammen eine Bindung bedeuten umgewandelt werden. Das gelingt z.B. durch Einwirkung einer der angegebenen Basen, z.B. NaOH oder NaH, in einem der angegebenen Lösungsmittel, z.B. THF Dioxan oder DMSO, bei Temperaturen zwischen 0 und 150°.

Weiterhin kann man in einer Verbindung der Formel I einen oder mehrere der Reste R², R³, R⁵ und/oder R⁶ in andere Reste R², R³, R⁵ und/oder R⁶ umwandeln.

Beispielsweise ist es möglich, daß man eine Hydroxygruppe alkyliert oder eine Carbamoylgruppe (z.B. mit POCl₃) zu einer Cyangruppe dehydratisiert.

Eine OH-Gruppe kann durch Behandeln mit alkylierenden Mitteln in die entsprechende Alkoxygruppe umgewandelt werden. Als alkylierende Mittel eignen sich z.B. Verbindungen der Formeln A-Cl, A-Br oder A-J oder entsprechende Schwefelsäure- oder Sulfonsäureester wie Methylchlorid, -bromid, -jodid, Dimethylsulfat, Methyl-p-toluolsulfonat. Ferner kann man z.B. eine Methylgruppe mit Formaldehyd in Gegenwart von Ameisensäure einführen. Die Alkylierung wird zweckmäßig in Gegenwart oder Abwesenheit eines der genannten inerten Lösungsmittel, z.B. DMF, bei Temperaturen zwischen etwa 0° und etwa 120° vorgenommen, wobei auch ein Katalysator zugegen sein kann, vorzugsweise eine Base wie Kalium-tert.-butylat oder NaH.

Eine Base der Formel I kann mit einer Säure in das zugehörige Säureadditionssalz übergeführt werden. Für diese Umsetzung kommen insbesondere Säuren in Frage, die physiologisch unbedenkliche Salze liefern. So können anorganische Säuren verwendet werden, z.B. Schwefelsäure, Salpetersäure, Halogenwasserstoffsäuren wie Chlorwasserstoffsäure oder Bromwasserstoffsäure, Phosphorsäure wie Orthophosphorsäure, Sulfaminsäure, ferner organische Säuren, insbesondere aliphatische, alicyclische, araliphatische, aromatische oder heterocyclische ein- oder mehrbasige Carbon-, Sulfon- oder Schwefelsäuren, z.B. Ameisensäure, Essigsäure, Propionsäure, Pivalinsäure, Diethylessigsäure, Malonsäure, Bernsteinsäure, Pimelinsäure, Fumarsäure, Maleinsäure, Milchsäure, Weinsäure. Äpfelsäure, Benzoesäure. Salicylsäure, 2- oder 3-Phenylpropionsäure, Citronensäure, Gluconsäure, Ascorbinsäure, Nicotinsäure, Isonicotinsäure, Methan- oder Ethansulfonsäure, Ethandisulfonsäure, 2-Hydroxyethansulfonsäure, Benzolsulfonsäure, p-Toluolsulfonsäure, Naphthalin-mono- und disulfonsäuren, Laurylschwefelsäure. Salze mit physiologisch nicht unbedenklichen Säuren, z.B. Pikrate können zur Aufreinigung der Verbindungen der Formel I verwendet werden.

Die Verbindungen der Formel I können ein oder mehrere chirale Zentren besitzen. Sie können daher bei ihrer Herstellung als Racemate oder, falls optisch aktive Ausgangsstoffe verwendet werden, auch in optisch aktiver Form erhalten werden. Weisen die Verbindungen zwei oder mehr chirale Zentren auf, dann können sie bei der Synthese als Gemische von Racematen anfallen, aus denen man die einzelnen Racemate, beispielsweise durch Umkristallisieren aus inerten Lösungsmitteln, in reiner Form isolieren kann. So haben z.B. Verbindungen der Formel I, worin R³ = OH und R⁴ = H ist, drei chirale Zentren; bei der Herstellung durch Reaktion von II mit III entstehen jedoch ganz überwiegend nur zwei Racemate, beide mit trans-Stellung der R⁵-Z-gruppe und des Substituenten R³ = OH. Erhaltene Racemate können, falls erwünscht, nach sich bekannten Methoden mechanisch, chemisch oder biochemisch in ihre Enantiomeren getrennt werden. So können aus dem Racemat durch Umsetzung mit einem optisch aktiven Trennmittel Diastereomere gebildet werden. Als Trennmittel für basische Verbindungen der Formel I eignen sich z.B. optisch aktive Säuren, wie die D- und L-Formen von Weinsäure, Dibenzoylweinsäure, Diacetylweinsäure, Camphansäure, Camphersulfonsäuren, Mandelsäure, Äpfelsäure oder Milchsäure. Carbinole (I, R³ = OH) können ferner mit Hilfe chiraler Acylierungsreagenzien, z.B. D- oder L-α-Methylbenzylisocyanat, verestert und dann getrennt werden (vgl. EP-A1-120428). Die verschiedenen Formen der Diastereomeren können in an sich bekannter Weise, z.B. durch fraktionierte Kristallisation, getrennt, und die Enantiomeren der Formel I können in an sich bekannter Weise aus den Diastereomeren in Freiheit gesetzt werden. Enantiomerentrennungen gelingen ferner durch Chromatographie an optisch-aktiven Trägermaterialien.

Die Verbindungen der Formel I und ihre physiologisch unbedenklichen Salze können zur Herstellung pharmazeutischer Zubereitungen verwendet werden, insbesondere auf nicht-chemischem Wege. Hierbei können sie zusammen mit mindestens einem festen, flüssigen und/oder halbflüssigen Träger- oder Hilfsstoff und gegebenenfalls in Kombination mit einem oder mehreren weiteren Wirkstoff(en) in eine geeignete Dosierungsform gebracht werden.

Gegenstand der Erfindung sind ferner Mittel, insbesondere pharmazeutische Zubereitungen, enthaltend mindestens eine Verbindung der Formel I und/oder eines ihrer physiologisch unbedenklichen Salze.

Diese Zubereitungen können als Arzneimittel in der Human- oder Veterinärmedizin verwendet werden. Als Trägerstoffe kommen organische oder anorganische Substanzen in Frage, die sich für die neterale (z.B. orale), parenterale oder topische Applikation eignen und mit den neuen Verbindungen nicht reagieren, beispielsweise Wasser, pflanzliche Öle, Benzylalkohole, Polyethylenglykole, Glycerintriacetat, Gelatine, Kohlehydrate wie Lactose oder Stärke, Magnesiumstearat, Talk, Lanolin, Vaseline. Zur oralen Anwendung dienen insbesondere Tabletten, Dragees, Kapseln, Sirupe, Säfte oder Tropfen, zur rektalen Anwendung Suppositorien, zur parenteralen Anwendung Lösungen, vorzugsweise ölige oder wässerige Lösungen, ferner Suspensionen, Emulsionen oder Implantate, für die topische Anwendung Salben, Cremes, Pasten, Lotionen, Gele, Sprays, Schäume, Aerosole, Lösungen (z.B. Lösungen in Alkoholen wie Ethanol oder Iospropanol, Acetonitril, DMF, Dimethylacetamid, 1,2-Propandiol oder deren Gemischen untereinander und/oder mit Wasser) oder Puder. Die neuen Verbindungen können auch lyophilisiert und die erhaltenen Lyophilisate z.B. zur Herstellung von Injektionspräparaten verwendet werden. Insbesondere für die topische Anwendung kommen auch liposomale Zubereitungen in Betracht. Die angegebenen Zubereitungen können sterilisiert sein und/oder Hilfsstoffe wie Gleit-, Konservierungs-, Stabilisierungs- und/oder Netzmittel, Emulgatoren, Salze zur Beeinflussung des osmotischen Druckes, Puffersubstanzen, Farb-, Geschmacks- und/oder Aromastoffe enthalten. Sie können, falls erwünscht, auch einen oder mehrere weitere Wirkstoffe enthalten, z.B. ein oder mehrere Vitamine.

Die Verbindungen der Formel I und ihre physiologisch unbedenklichen Salze können an Menschen oder Tiere, insbesondere Säugetiere wie Affen, Hunde, Katzen, Ratten oder Mäuse verabreicht und bei der therapeutischen Behandlung des menschlichen oder tierischen Körpers sowie bei der Bekämpfung von Krankheiten verwendet werden, insbesondere bei der Therapie und/oder Prophylaxe von Störungen des cardiovasculären Systems, insbesondere dekompensierter Herzinsuffizienz, Angina pectoris, Arrhythmie, peripheren oder cerebralen Gefäßerkrankungen, sowie Krankheitszuständen, die mit Bluthochdruck verbunden sind, ferner von Erkrankungen, die mit Veränderungen der nicht-vaskulären Muskulatur verbunden sind, z.B. Asthma, Inkontinenz der Harnblase.

Dabei werden die erfindungsgemäßen Substanzen in der Regel in Analogie zu bekannten Antianginosa bzw. Blutdrucksenkern, z.B. Nicorandil oder Cromakalim verabreicht, vorzugsweise in Dosierungen zwischen etwa 0,01 und 5 mg, insbesondere zwischen 0,02 und 0,5 mg pro Dosierungseinheit. Die tägliche Dosierung liegt vorzugsweise zwischen etwa 0,0001 und 0,1, insbesondere zwischen 0,0003 und 0,01 mg/kg Körpergewicht. Die spezielle Dosis für jeden bestimmten Patienten hängt jedoch von den verschiedensten Faktoren ab, beispielsweise von der Wirksamkeit der eingesetzten speziellen Verbindung, vom Alter, Körpergewicht, dem allgemeinen Gesundheitszustand, Geschlecht, von der Kost, vom Verabfolgungszeitpunkt und -weg, von der Ausscheidungsgeschwindigkeit, Arzneistoffkombination und Schwere der jeweiligen Erkrankung, welcher die Therapie gilt. Die orale Applikation ist bevorzugt.

Die Verbindungen der Formel I und ihre Salze eignen sich, insbesondere bei topischer Anwendung, ferner zur Behandlung der Alopecia areata. Hierfür werden insbesondere pharmazeutische Zubereitungen verwendet, die zur topischen Behandlung der Kopfhaut geeignet und die oben genannt sind. Sie erhalten etwa 0,005 bis 10, bevorzugt 0,5 bis 3 Gew.% mindestens einer Verbindung der Formel I und/oder mindestens einer ihrer Salze. Im übrigen können diese Verbindungen gegen Alopezie in Analogie zu den Angaben in der WO 88/00822 verwendet werden.

In den nachfolgenden Beispielen bedeutet "übliche Aufarbeitung":
Man gibt, falls erforderlich, Wasser hinzu, extrahiert mit einem organischen Lösungsmittel wie Ethylacetat, trennt ab, trocknet die organische Phase über Natriumsulfat, filtriert, dampft ein und reinigt durch Chromatographie und/oder Kristallisation.

Vor- und nachstehend sind alle Temperaturen in °C angegeben.

### Beispiel 1

Ein Gemisch von 23,1 g 2-Methoxymethyl-2-methyl-3,4-epoxy-6-cyan-chroman, 9,5 g 1H-2-Pyridon, 12 ml Pyridin und 600 ml Ethanol wird 72 Std. gekocht. Man destilliert etwa 300 ml ab, kühlt ab, filtriert, dampft ein und arbeitet wie üblich auf. Man erhält 2-Methoxymethyl-2-methyl-4-(1,2-dihydro-2-oxo-1-pyridyl)-6-cyan-3-chromanol, Stereoisomerengemisch ("A"), das chromatographisch in Isomer "A1" (CH₃ und OH in cis-Stellung; F. 216-218°) und Isomer "A2" (CH₃ und OH in trans-Stellung; F. 210-212°) getrennt werden kann (Kieselgel; Ethylacetat/Methanol).

Das Ausgangsmaterial wird wie folgt erhalten:
a) Ein Gemisch von 50 g 3-Acetyl-4-hydroxy-benzonitril, 70 ml Methoxy-2-propanon, 6 ml Pyrrolidin und 300 ml Toluol wird 3 Std. am Wasserabscheider gekocht. Man dampft ein, arbeitet wie üblich auf (Ethylacetat/verd. Salzsäure) und erhält 2-Methoxymethyl-2-methyl-6-cyan-4-chromanon, F. 79-81°.
b) Eine Lösung von 31,4 g des vorstehenden Chromanons in 600 ml Methanol wird unter Rühren portionsweise mit 7 g NaBH₄ versetzt. Man rührt noch 1 Std., dampft ein, arbeitet wie üblich auf und erhält 2-Methoxymethyl-2-methyl-6-cyan-4-chromanol, Stereoisomerengemisch, Kp. 160°/0,133 mbar (Luftbadtemperatur, Kugelrohr).
c) Eine Lösung von 13 g des vorstehenden Chromanols und 0,8 g p-Toluolsulfonsäure in 100 ml Toluol wird 3 Std. am Wasserabscheider gekocht. Man dampft ein, löst den Rückstand in Dichlormethan, chromatographiert an Kieselgel mit Petrolether/Ether und erhält 2-Methoxymethyl-2-methyl-6-cyan-4-chromen, Kp. 135°/0,133 mbar (Luftbadtemperatur, Kugelrohr).
d) Zu einer Lösung von 13 g des vorstehenden Chromens in 50 ml Dichlormethan tropft man unter Rühren und Eiskühlung eine Lösung von 21,1 g m-Chlorperbenzoesäure in 100 ml Dichlormethan. Man rührt 3,5 Std. bei 20°, wobei nach 3 Std. eine weitere Lösung von 5 g m-Chlorperbenzoesäure in 30 ml Dichlormethan zugetropft wird, wäscht mit vedünnter Natronlauge und arbeitet weiter wie üblich auf. Man erhält 2-Methoxymethyl-2-methyl-3,4-epoxy-6-cyan-4-chroman als Isomerengemisch, das chromatographisch (Kieselgel; Petrolether/Ether 4:6) in zwei Isomere (F. 88-90° bzw. Öl) getrennt werden kann.

Man erhält aus diesen Epoxiden mit 4-Hydroxy-1H-2-pyridon:
2-Methoxymethyl-2-methyl-4-(1,2-dihydro-2-oxo-4-pyridyl-oxy)-6-cyan-3-chromanol

### Beispiel 2

Eine Lösung von 2,96 g 2-Methoxymethyl-2-methyl-4-brom-6-cyan-chroman (Stereoisomerengemisch; erhältlich aus 2-Methoxymethyl-2-methyl-6-cyan-4-chromanol und PBr₃ in Toluol bei 20°) und 2 g 1H-2-Pyridon in 70 ml DMSO wird mit 1,2 g 80%igem NaH versetzt und 3 Tage bei 20° gerührt. Nach üblicher Aufarbeitung erhält man 2-Methoxymethyl-2-methyl-4-(1,2-dihydro-2-oxo-1-pyridyl)-6-cyan-chroman, Isomerengemisch.

### Beispiel 3

Ein Gemisch von 10 g "A", 3 g NaOH und 350 ml Dioxan wird 20 Min. gekocht. Man kühlt ab, filtriert, dampft das Filtrat ein und erhält 2-Methoxymethyl-2-methyl-4-(1,2-dihydro-2-oxo-1-pyridyl)-6-cyan-chromen ("B"), F. 160-162°.

Analog erhält man durch Dehydratisierung der entsprechenden 3-Chromanole:
2-Methoxymethyl-2-methyl-4-(1,2-dihydro-2-oxo-4-pyridyl-oxy)-6-cyan-3-chromen.

### Beispiel 4

Ein Gemisch von 1 g 2-Methyl-2-(2-tetrahydropyranyl-oxy-methyl)-4-(1,2-dihydro-2-oxo-1-pyridyl)-6-cyan-3-chroman [erhältlich aus 1-(2-Tetrahydropyranyl)-2-propanon über 2-Methyl-2-(2-tetrahydropyranyl-oxy-methyl)-6-cyan-4-chromanon, 2-Methyl-2-(2-tetrahydropyranyl-oxy-methyl)-6-cyan-4-chromanol, 2-Methyl-2-(2-tetrahydropyranyl-oxy-methyl)-6-cyan-3-chromen und das entsprechende 3,4-Epoxid] und 15 ml 10%iger methanolischer HCl-Lösung wird 15 Min. gekocht. Nach Abkühlen und üblicher Aufarbeitung erhält man 2-Hydroxymethyl-2-methyl-4-(1,2-dihydro-2-oxo-1-pyridyl)-6-cyan-3-chromen.

### Beispiel 5

Ein Gemisch von 2,98 g 2-Methoxymethyl-2-methyl-4-(1,2-dihydro-2-oxo-1-pyridyl)-6-carbamoyl-chroman, 2 ml POCl₃ und 200 ml 1,2-Dichlorethan wird 45 Min. gekocht. Nach dem Abkühlen und üblicher Aufarbeitung erhält man 2-Methoxymethyl-2-methyl-4-(1,2-dihydro-2-oxo-1-pyridyl)-6-cyan-chroman.

Die nachstehenden Beispiele betreffen pharmazeutische Zubereitungen, welche Verbindungen der Formel I und/oder deren physiologisch unbedenkliche Salze enthalten.

### Beispiel 1: Tabletten

Ein Gemisch von 0,2 kg "B", 136,3 kg Calciumhydrogenphosphat, 15 kg Maisstärke, 10 kg mikrokristalliner Cellulose, 5,5 kg unlöslichem Polyvinylpyrrolidon (PVP), 1,5 kg hochdispersem Siliciumdioxid und 1,5 kg Magnesiumstearat wird in üblicher Weise zu Tabletten gepreßt. Jede 170-mg-Tablette enthält 0,2 mg Wirkstoff.

### Beispiel 2: Dragees

Analog Beispiel 1, jedoch ohne den Zusatz von PVP, werden Tabletten gepreßt, die anschließend in üblicher Weise mit einem Überzug aus Saccharose, Maisstärke, Talk, Tragant und Farbstoff überzogen werden.

### Beispiel 3: Lacktabletten

Aus 0,2 kg "B", 151,3 kg Lactose, 10 kg mikrokristalliner Cellulose, 5,5 kg unlöslichem PVP, 1,5 kg hochdispersem Siliciumdioxid und 1,5 kg Calciumstearat werden Tablettenkerne (170 mg) gepreßt, die anschließend in üblicher Weise lackiert werden, so daß jede Lacktablette mit 3,922 mg eines Lackes überzogen ist, der aus 2,2 mg Hydroxypropylmethylcellulose, 0,53 mg Polyethylenglykol 400, 0,85 mg Titandioxid, 0,12 mg Eisen(III)-oxid (gelb), 0,002 mg Eisen(III)-oxid (rot) und 0,22 mg Silikonöl besteht.

### Beispiel 4: Kapseln

Man bereitet ein Granulat aus 10 g "A", 27,5 kg Lactose, 0,35 kg Hydroxypropylmethylcellulose und 0,7 kg Maisstärke, mischt dieses mit 0,15 kg hochdispersem Siliciumdioxid und 0,3 kg Magnesiumstearat und füllt das Gemisch in üblicher Weise in Hartgelatinekapseln, so daß jede Kapsel 0,1 mg Wirkstoff enthält.

### Beispiel 5: Ampullen

Ein Lösung von 10 g "A2" in 70 l 1,2-Propandiol wird mit zweifach destilliertem Wasser auf 100 l aufgefüllt, steril filtriert, und in 1-ml-Ampullen abgefüllt, die dann steril verschlossen werden. Jede Ampulle enthält 0,1 mg Wirkstoff.

## Patentansprüche (Patentansprüche für folgende(n) Vertragsstaat(en): AT, BE, CH, DE, DK, FR, GB, GR, IT, LI, LU, NL, SE)

1. Chromanderivate der Formel I 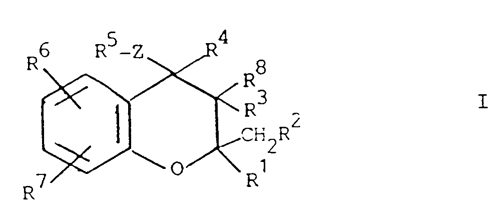 worin
R¹ A,
R² OA,
R³ OH,
R⁴ H oder
R³ und R₄ zusammen auch eine Bindung,
R⁵ einen unsubstituierten oder einfach durch A substituierten Oxo-dihydro-pyridyl-rest,
R⁶ CN
R⁷ und R⁸ jeweils H,
Z O, NH oder eine Bindung,
A Alkyl mit 1-6 C-Atomen,
bedeuten,
sowie deren Salze.

2. a) 2-Methoxymethyl-2-methyl-4-(1,2-dihydro-2-oxo-1-pyridyl)-6-cyan-3-chromanol;
b) 2-Methoxymethyl-2-methyl-4-(1,2-dihydro-2-oxo-1-pyridyl)-6-cyan-3-chromen.

3. Verfahren zur Herstellung von Chromanderivaten der Formel I gemäß Anspruch 1, dadurch gekennzeichnet, daß man ein Chroman der Formel II 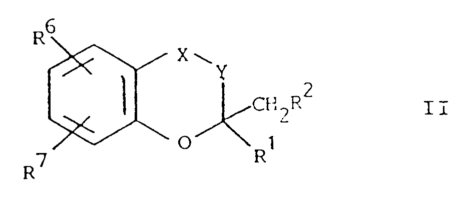 worin
X-Y 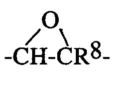 oder -CHE-CR³-R⁸- und
E Cl, Br, J oder eine reaktionsfähig veresterte OH-Gruppe bedeuten und
R¹, R², R³, R⁶, R⁷ und R⁸ die bei Formel I angegebenen Bedeutungen haben,
mit einer Verbindung der Formel III
R⁵-Z-H III
worin
R⁵ und Z die bei der Formel I angegebenen Bedeutungen haben,
umsetzt,
und/oder daß man eine Verbindung der Formel I, worin R³ OH und R⁴ H bedeutet, dehydratisiert und/oder daß man in einer Verbindung der Formel I einen oder mehrere der Reste R², R³, R⁵ und/oder R⁶ in andere Reste R², R³, R⁵ und/oder R⁶ umwandelt und/oder daß man eine basische Verbindung der Formel I durch Behandeln mit einer Säure in eines ihrer Säureadditionssalze umwandelt.

4. Verfahren zur Herstellung pharmazeutischer Zubereitungen, dadurch gekennzeichnet, daß man eine Verbindung der Formel I und/oder eines ihrer physiologisch unbedenklichen Salze zusammen mit mindestens einem festen, flüssigen oder halbflüssigen Träger- oder Hilfsstoff in eine geeignete Dosierungsform bringt.

5. Pharmazeutische Zubereitung, gekennzeichnet durch einen Gehalt an mindestens einer Verbindung der Formel I und/oder einem ihrer physiologisch unbedenklichen Salze.

6. Verwendung von Verbindungen der Formel I zur Herstellung eines Arzneimittels.

## Patentansprüche (Patentansprüche für folgende(n) Vertragsstaat(en): ES)

1. Verfahren zur Herstellung von Chromanderivaten der Formel I 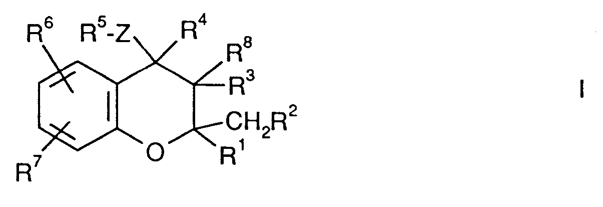 worin
R¹ A,
R² OA,
R³ OH,
R⁴ H oder
R³ und R⁴ zusammen auch eine Bindung,
R⁵ einen unsubstituierten oder einfach durch A substituierten Oxo-dihydro-pyridyl-rest,
R⁶ CN
R⁷ und R⁸ jeweils H,
Z O, NH oder eine Bindung,
A Alkyl mit 1-6 C-Atomen,
bedeuten,
sowie von deren Salzen,
dadurch gekennzeichnet, daß man ein Chroman der Formel II 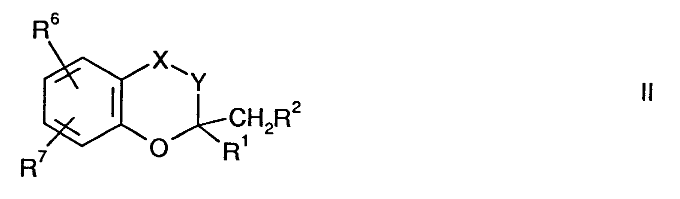 worin
X-Y 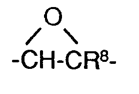 oder -CHE-CR³-R⁸- und
E Cl, Br, J oder eine reaktionsfähig veresterte OH-Gruppe bedeuten und
R¹, R², R³, R⁶, R⁷ und R⁸ die bei Formel I angegebenen Bedeutungen haben,
mit einer Verbindung der Formel III
R⁵-Z-H III
worin
R⁵ und Z die bei der Formel I angegebenen Bedeutungen haben,
umsetzt,
und/oder daß man eine Verbindung der Formel I, worin R³ OH und R⁴ H bedeutet, dehydratisiert und/oder daß man in einer Verbindung der Formel I einen oder mehrere der Reste R², R³, R⁵ und/oder R⁶ in andere Reste R², R³, R⁵ und/oder R⁶ umwandelt und/oder daß man eine basische Verbindung der Formel I durch Behandeln mit einer Säure in eines ihrer Säureadditionssalze umwandelt.

2. Verfahren zur Herstellung von
a) 2-Methoxymethyl-2-methyl-4-(1,2-dihydro-2-oxo-1-pyridyl)-6-cyan-3-chromanol,
b) 2-Methoxymethyl-2-methyl-4-(1,2-dihydro-2-oxo-1-pyridyl)-6-cyan-3-chromen
gemäß Anspruch 1.

3. Verfahren zur Herstellung pharmazeutischer Zubereitungen, dadurch gekennzeichnet, daß man eine Verbindung der Formel I und/oder eines ihrer physiologisch unbedenklichen Salze zusammen mit mindestens einem festen, flüssigen oder halbflüssigen Träger- oder Hilfsstoff in eine geeignete Dosierungsform bringt.

## Claims (Claims for the following Contracting State(s): AT, BE, CH, DE, DK, FR, GB, GR, IT, LI, LU, NL, SE)

1. Chroman derivatives of the formula I 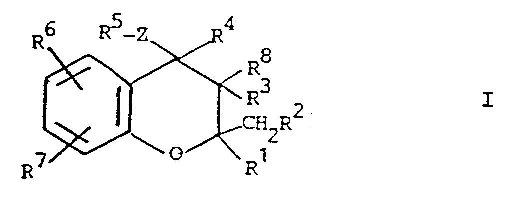 in which
R¹ is A,
R² is OA,
R³ is OH,
R⁴ is H or
R³ and R₄ [sic] together are also a bond,
R⁵ is an oxodihydropyridyl radical which is unsubstituted or monosubstituted by A,
R⁶ is CN,
R⁷ and R⁸ are each H,
Z is O, NH or a bond,
A is alkyl having 1-6 C atoms,
and their salts.

2. a) 2-Methoxymethyl-2-methyl-4-(1,2-dihydro-2-oxo-1-pyridyl)-6-cyano-3-chromanol;
b) 2-Methoxymethyl-2-methyl-4-(1-benzyl-1,2-dihydro-2-oxo-1-pyridyl)-6-cyano-3-chromene.

3. Process for the preparation of chroman derivatives of the formula I according to Claim 1, characterized in that a chroman of the formula II 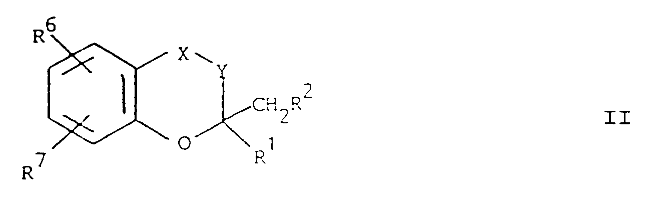 in which
X-Y is 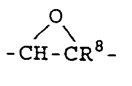 or -CHE-CR³R⁸- and
E is Cl, Br, I or a reactively esterified OH group and
R¹, R², R³, R⁶, R⁷ and R⁸ have the meanings given in formula I, is reacted with a compound of the formula III
R⁵-Z-H III
in which
R⁵ and Z have the meanings indicated in the formula I, and/or in that a compound of the formula I, in which R³ is OH and R⁴ is H, is dehydrated and/or in that in a compound of the formula I, one or more of the radicals R², R³, R⁵ and/or R⁶ R⁷ are converted into other radicals R², R³, R⁵ and/or R⁶ and/or in that a basic compound of the formula I is converted into one of its acid addition salts by treating with an acid.

4. Process for the production of pharmaceutical preparations, characterized in that a compound of the formula I and/or one of its physiologically acceptable salts is brought into a suitable form for administration together with at least one solid, liquid or semi-liquid excipient or auxiliary.

5. Pharmaceutical preparation which contains at least one compound of the formula I and/or one of its physiologically acceptable salts.

6. Use of compounds of the formula I for the production of a medicament.

## Claims (Claims for the following Contracting State(s): ES)

1. Process for the preparation of chroman derivatives of the formula I 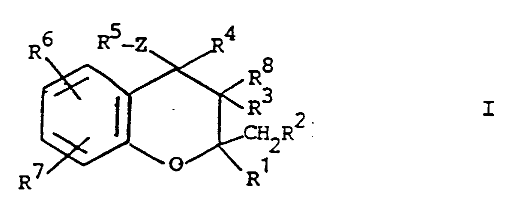 in which
R¹ is A,
R² is OA,
R³ is OH,
R⁴ is H or
R³ and R⁴ together are also a bond,
R⁵ is an oxodihydropyridyl radical which is unsubstituted or monosubstituted by A,
R⁶ is CN,
R⁷ and R⁸ are each H,
Z is O, NH or a bond,
A is alkyl having 1-6 C atoms,
and of their salts, characterized in that a chroman of the formula II 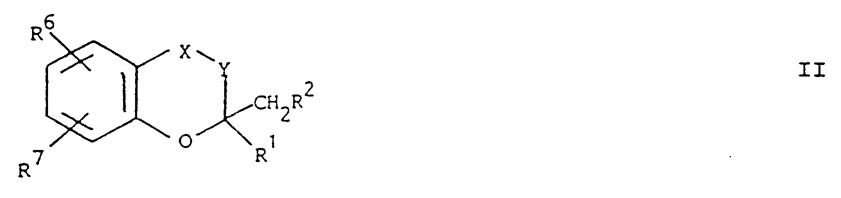 in which
X-Y is 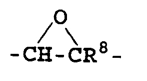 or -CHE-CR³R⁸- and
E is Cl, Br, I or a reactively esterified OH group and
R¹, R², R³, R⁶, R⁷ and R⁸ have the meanings given in formula I, is reacted with a compound of the formula III
R⁵-Z-H III
in which
R⁵ and Z have the meanings indicated in the formula I, and/or in that a compound of the formula I, in which R³ is OH and R⁴ is H, is dehydrated and/or in that in a compound of the formula I, one or more of the radicals R², R³, R⁵ and/or R⁶ are converted into other radicals R², R³, R⁵ and/or R⁶ and/or in that a basic compound of the formula I is converted into one of its acid addition salts by treating with an acid.

2. Process for the preparation of
a) 2-Methoxymethyl-2-methyl-4-(1,2-dihydro-2-oxo-1-pyridyl)-6-cyano-3-chromanol,
b) 2-Methoxymethyl-2-methyl-4-(1-benzyl-1,2-dihydro-2-oxo-1-pyridyl)-6-cyano-3-chromene according to Claim 1.

3. Process for the production of pharmaceutical preparations, characterized in that a compound of the formula I and/or one of its physiologically acceptable salts is brought into a suitable form for administration together with at least one solid, liquid or semi-liquid excipient or auxiliary.

## Revendications (Revendications pour l'(les) Etat(s) contractant(s) suivant(s): AT, BE, CH, DE, DK, FR, GB, GR, IT, LI, LU, NL, SE)

1. Dérivés de chromane de formule I 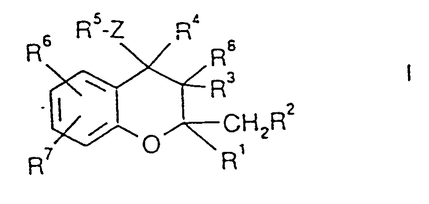 dans laquelle
R¹ représente A,
R² représente OA,
R³ représente OH,
R⁴ représente H ou
R³ et R⁴ représentent ensemble aussi une liaison,
R⁵ représente un reste oxo-dihydro-pyridyle non-substitué ou substitué une fois par A,
R⁶ représente CN,
R⁷ et R⁸ représentent chacun H,
Z représente O, NH ou une liaison,
A représente un alkyle de 1 à 6 atomes de C,
ainsi que leurs sels.

2. a) 2-méthoxyméthyl-2-méthyl-4-(1,2-dihydro-2-oxo-1-pyridyl)-6-cyano-3-chromanol ;
b) 2-méthoxyméthyl-2-méthyl-4-(1,2-dihydro-2-oxo-1-pyridyl)-6-cyano-3-chromène.

3. Procédé de préparation de dérivés de chromane de formule I selon la revendication 1, caractérisé en ce qu'on fait réagir un chromane de formule II 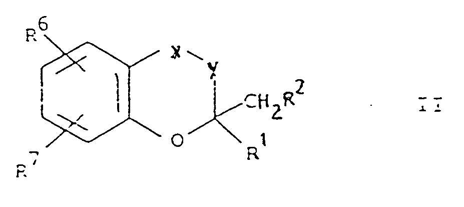 dans laquelle
X-Y représente 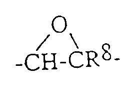 ou -CHE-CR³-R⁸- et
E représente Cl, Br, I ou un groupe OH estérifié réactif et
R¹, R², R³, R⁶, R⁷ et R⁸ ont les significations données pour la formule I
avec un composé de formule III
R⁵-Z-H III
dans laquelle R⁵ et Z ont les significations données pour la formule I
et/ou on déshydrate un composé de formule I dans laquelle R³ représente OH et R⁴ représente H, et/ou on transforme dans un composé de formule I un ou plusieurs des restes R², R³, R⁵ et/ou R⁶ et/ou on transforme un composé basique de formule I en l'un de ses sels d'addition d'acide par traitement avec un acide.

4. Procédé de production de préparations pharmaceutiques caractérisé en ce qu'on met sous une forme de dosage appropriée un composé de formule I et/ou un de ses sels physiologiquement non-toxiques en présence d'au moins un véhicule ou additif solide, liquide ou semi-liquide.

5. Préparation pharmaceutique caractérisée par une teneur en au moins un composé de formule I et/ou un de ses sels physiologiquement non-toxiques.

6. Utilisation des composés de formule I pour produire un médicament.

## Revendications (Revendications pour l'(les) Etat(s) contractant(s) suivant(s): ES)

1. Procédé de production de dérivés de chromane de formule I 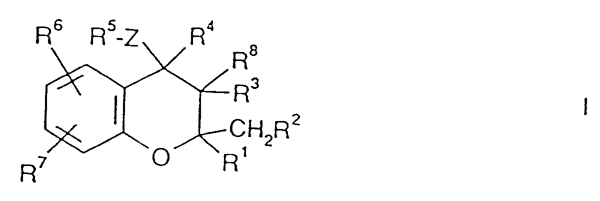 dans laquelle
R¹ représente A,
R² représente OA,
R³ représente OH,
R⁴ représente H ou
R³ et R⁴ représentent ensemble aussi une liaison,
R⁵ représente un reste oxo-dihydro-pyridyle non-substitué ou substitué une fois par A,
R⁶ représente CN,
R⁷ et R⁸ représentent chacun H,
Z représente O, NH ou une liaison,
A représente un alkyle de 1 à 6 atomes de C,
ainsi que leurs sels,
caractérisé en ce qu'on fait réagir un chromane de formule II 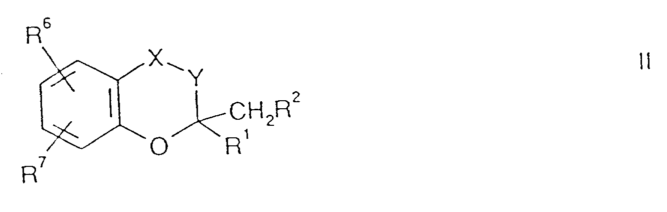 dans laquelle
X-Y représente 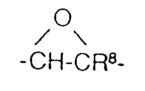 ou -CHE-CR³-R⁸- et
E représente Cl, Br, I ou un groupe OH estérifié réactif et
R¹, R², R³, R⁶, R⁷ et R⁸ ont les significations données pour la formule I
avec un composé de formule III
R⁵-Z-H III
dans laquelle R⁵ et Z ont les significations données pour la formule I
et/ou on déshydrate un composé de formule I dans laquelle R³ représente OH et R⁴ représente H, et/ou on transforme dans un composé de formule I un ou plusieurs des restes R², R³, R⁵ et/ou R⁶ et/ou on transforme un composé basique de formule I en l'un de ses sels d'addition d'acide par traitement avec un acide.

2. Procédé de production de
a) 2-méthoxyméthyl-2-méthyl-4-(1,2-dihydro-2-oxo-1-pyridyl)-6-cyano-3-chromanol,
b) 2-méthoxyméthyl-2-méthyl-4-(1,2-dihydro-2-oxo-1-pyridyl)-6-cyano-3-chromène
selon la revendication 1.

3. Procédé de production de préparations pharmaceutiques caractérisé en ce qu'on met sous une forme de dosage appropriée un composé de formule I et/ou un de ses sels physiologiquement non-toxiques en présence d'au moins un véhicule ou additif solide, liquide ou semi-liquide.
